# EUROPEAN PATENT APPLICATION

(11) **EP 4 235 680 A2**
(43) Date of publication of application: **30.08.2023**
(21) Application number: 23164744.7
(22) Date of filing: 11.10.2016
(51) Int. Cl.: G16B 50/50

(54) **METHOD AND APPARATUS FOR COMPACT REPRESENTATION OF BIOINFORMATICS DATA**

(62) Divisional of application: 16791320.1
(71) Applicant: Genomsys SA, 1015 Lausanne (CH)
(72) Inventor: ZOIA, Giorgio, 1007 Lausanne (CH); RENZI, Daniele, 1004 Lausanne (CH)
(74) Representative: Metroconsult Srl

(57) **Abstract**

A computer-implemented method for the compression of genome sequence data produced by a sequencing machine, said genome sequence data comprising reads of sequences of nucleotides, said method comprising the steps of: aligning said reads to one or more reference sequences thereby creating aligned reads, classifying said aligned reads according to matching accuracy degrees with the one or more reference sequences thereby creating classes of aligned reads; encoding said classified and aligned reads as a multiplicity of syntax elements, wherein encoding said classified aligned reads as a multiplicity of syntax elements comprises selecting said syntax elements according to said classes of aligned reads.

## Description

### TECHNICAL FIELD

This disclosure provides a novel method of representation of genome sequencing data which reduces the utilized storage space and improves access performance by providing new functionality that are not available with known prior art methods of representation.

### BACKGROUND

An appropriate representation of genome sequencing data is fundamental to enable efficient genomic analysis applications such as genome variants calling and all other analysis performed with various purposes by processing the sequencing data and metadata.
Human genome sequencing has become affordable by the emergence of high-throughput low cost sequencing technologies. Such opportunity opens new perspectives in several fields ranging from the diagnosis and treatment of cancer to the identification of genetic illnesses, from pathogen surveillance for the identification of antibodies to the creation of new vaccines, drugs and customization of personalized treatments.
Hospitals, genomic analysis providers, bioinformatics and large biological data storage centers are looking for affordable, fast, reliable and interconnected genomic information processing solutions which could enable scaling genomic medicine to a world-wide scale. Since one of the bottleneck in the sequencing process has become data storage, methods for representing genome sequencing data in a compressed form are increasingly investigated.

The most used genome information representations of sequencing data are based on zipping FASTQ and SAM formats. The objective is to compress the traditionally used file formats (respectively FASTQ and SAM for non-aligned and aligned data). Such files are constituted by plain text characters and are compressed, as mentioned above, by using general purpose approaches such as LZ (from Lempel and Ziv, the authors who published the first versions) schemes (the well-known zip, gzip etc). When general purpose compressors such as gzip are used, the result of compression is usually a single blob of binary data. The information in such monolithic form results quite difficult to archive, transfer and elaborate particularly when like in the case of high throughput sequencing the volume of data are extremely large. The BAM format is characterized by poor compression performance due to the focus on compression of the inefficient and redundant SAM format rather than on extracting the actual genomic information conveyed by SAM files and due to the adoption of general purpose text compression algorithms such as gzip rather than exploiting the specific nature of each data source (the genomic data itself).
A more sophisticated approach to genomic data compression that is less used, but more efficient than BAM is CRAM. CRAM provides more efficient compression for the adoption of differential encoding with respect to an existing reference (it partially exploits the data source redundance), but it still lacks features such as incremental updates, support for streaming and selective access to specific classes of compressed data.

These approaches generate poor compression ratios and data structures that are difficult to navigate and manipulate once compressed. Downstream analysis can be very slow due to the necessity of handling large and rigid data structures even to perform simple operation or to access selected regions of the genomic dataset. CRAM relies on the concept of the CRAM record. Each CRAM record encodes a single mapped or unmapped reads by encoding all the elements necessary to reconstruct it.

CRAM has the following drawbacks:
1. For CRAM, data indexing is out of the scope of the specification (see section 12 of CRAM specification v 3.0) and it's implemented as a separate file. Conversely the approach of the invention described in this document employs a data indexing method that is integrated with the encoding process and indexes are embedded in the encoded bit stream.
2. In CRAM all core data blocks can contain any type of mapped reads (perfectly matching reads, reads with substitutions only, reads with insertions or deletions (also referred to as "indels")). There is no notion of classification and grouping of reads in classes according to the result of mapping with respect to a reference sequence
3. In the present invention there is no notion of record encapsulating each read because the data needed to reconstruct each read is scattered among several data containers called "layers". This enables more efficient access to set of reads with specific biological characteristics (e.g. reads with substitutions, but without "indels", or perfectly mapped reads) without the need of decoding each (block of) read(s) to inspect its features.
4. In a CRAM record each type of data is denoted by a specific flag. In the present invention there is no notion of flag denoting data because this is intrinsically defined by the "layer" the data belongs to. This implies a largely reduced number of symbols to be used and a consequent reduction of the information source entropy which results into a more efficient compression. This is due to the fact that the use of different "layers" enables the encoder to reuse the same symbol across each layer with different meanings. In CRAM each flag must always have the same meaning as there is no notion of contexts and each CRAM record can contain any type of data.
5. In CRAM substitutions, insertions and deletions are expressed according to different syntaxes, while the proposed approach uses a single alphabet and encoding for substitutions, insertions and deletions. This makes the encoding and decoding process simpler and produces a lower entropy source model which coding yields high compression bitstreams.

The present invention aims at compressing genomic sequences by organizing and partitioning data so that the redundant information to be coded is minimized and features such as selective access and support for incremental updates are enabled. One of the aspects of the presented approach is the definition of classes of data and metadata to be encoded separately and to be structured in different layers. The most important improvements of this approach with respect to existing methods consist in:
1. an increase of compression performance due to the reduction of the information source entropy constituted by providing an efficient model for each class of data or metadata;
2. the possibility of performing selective accesses to portions of the compressed data and metadata for any further processing purpose;
3. the possibility to incrementally (without the need of re-encoding) update encoded data and metadata with new sequencing data and/or metadata and/or new analysis results.

### BRIEF DESCRIPTION OF THE DRAWINGS

Figure 1 shows how the position of the mapped reads pairs are encoded in the pos layer as difference from the absolute position of the first mapped read.
Figure 2 shows how two reads in a pair can come from the two DNA strands.
Figure 3 shows how the reverse complement of read 2 will be encoded if strand 1 is used asreference.
Figure 4 shows the four possible combinations of reads composing a reads pair and the respective encoding in the rcomp layer.
Figure 5 shows how to calculate the pairing distance in case of constant reads length for three read pairs.
Figure 6 show how the pairing errors encoded in the pair layer enable the decoder to reconstruct the correct read pairing using the encoded MPPPD.
Figure 7 shows the encoding of a pairing distance when a read is mapped on a difference reference than its mate. In this case additional descriptors are added to the pairing distance. One is a signaling flag, the second is a reference identifier and then the pairing distance.
Figure 8 shows the encoding of N mismatches in a nmis layer.
Figure 9 shows a mapped read pair which presents substitutions with respect to a reference sequence.
Figure 10 shows how to calculate the positions of substitutions either as absolute or differential values.
Figure 11 shows how to calculate the symbols encoding substitutions types when no IUPAC codes are used. The symbols represent the distance - in a circular substitution vector - between the molecule present in the read and the one present on the reference at that position.
Figure 12 shows how to encode the substitutions into the snpt layer.
Figure 13 shows how to calculate substitution codes when IUPAC ambiguity codes are used.
Figure 14 shows how the snpt layer is encoded when IUPAC codes are used.
Figure 15 shows how for reads of class I the substitution vector used is the same as for class M with the addition of special codes for insertions of the symbols A, C, G, T, N.
Figure 16 shows some examples of encoding of mismatches and indels in case of IUPAC ambiguity codes. The substitution vector is much longer in this case and therefore the possible calculated symbols are more than in the case of five symbols.
Figure 17 shows a different source model for mismatches and indels where each layer contains the position of the mismatches or inserts of a single type. In this case no symbols are encoded for the mismatch or indel type.
Figure 18 shows an example of mismatches and indels encoding. When no mismatches or indels of a given type are present for a read, a 0 is encoded in the corresponding layer. The 0 acts as reads separator and terminator in each layer.
Figure 19 shows how a modification in the reference sequence can transform M reads in P reads. This operation can reduce the information entropy of the data structure especially in case of high coverage.
Figure 20 shows a genomic encoder 2010 according to one embodiment of this invention.
Figure 21 shows a genomic decoder 218 according to one embodiment of this invention.

### SUMMARY

The features of the independent claims below solve the problem of existing prior art solutions by providing a method for classification of genome sequences and a method for compression using said classification. In one aspect, a method for the classification of genome sequence data produced by a sequencing machine, said genome sequence data comprising sequences of nucleotides "bases", said classification being performed according to a reference sequence,
said method comprising the steps of:
   identifying class P sequences, comprising matching regions in the reference sequence without mismatches;
   identifying class N sequences, comprising matching regions in the reference sequence with a number of mismatches represented by positions where the sequencing machine was not able to call any "base";
   identifying class M sequences, comprising matching regions in the reference sequence with a number of mismatches represented by positions where the sequencing machine was not able to call any base or it called a different base than the reference sequence;
   identifying class I sequences, comprising the same mismatches of class M plus the presence of insertions or deletions;
   identifying class U sequences comprising all reads that do not find any valid mapping on the reference sequence.
   In another aspect, a method for the compression of genome sequence data produced by a sequencing machine, said genome sequence data comprising sequences of nucleotides,
said method comprising the steps of:
   aligning said reads to a reference sequence thereby creating aligned reads;
   classifying said aligned reads according to a multiplicity of matching accuracy degrees with the reference sequence thereby creating classes of aligned reads;
   encoding said aligned reads as layers of syntax elements;
   wherein said syntax elements are selected according to said classes of aligned reads.

In another aspect, a method for the decompression of a compressed genomic stream, said method comprising the steps of:
parsing said compressed genomic stream into genomic layers of syntax elements,
expanding said genomic layers into classified reads of sequences of nucleotides,
selectively decoding said classified reads of sequences of nucleotides with reference to one or more reference sequences so as to produce uncompressed reads of sequences of nucleotides.

A further aspect, a genomic encoder 2010 for the compression of genome sequence data 209, said genome sequence data 209 comprising reads of sequences of nucleotides, said genomic encoder 2010 comprising:
an aligner unit 201, configured to align said reads to one or more reference sequences thereby creating aligned reads,
a data classification unit 204, configured to classify said aligned reads according to matching accuracy degrees with the one or more reference sequences thereby creating classes of aligned reads;
one or more layers encoding units 205-207, configured to encode said classified aligned reads as layers of syntax elements by selecting said syntax elements according to said classes of aligned reads.

In another aspect, a genomic decoder 218 for the decompression of a compressed genomic stream 211 said genomic decoder 218 comprising:
parsing means 210, 212-214 configured to parse said compressed genomic stream into genomic layers of syntax elements 215,
one or more layer decoders 216-217, configured to decode the genomic layers into classified reads of sequences of nucleotides 2111,
genomic data classes decoders 213 configured to selectively decode said classified reads of sequences of nucleotides on one or more reference sequences so as to produce uncompressed reads of sequences of nucleotides.

### DETAILED DESCRIPTION

The genomic or proteomic sequences referred to in this invention include, for example, and not as a limitation, nucleotide sequences, Deoxyribonucleic acid (DNA) sequences, Ribonucleic acid (RNA), and amino acid sequences. Although the description herein is in considerable detail with respect to genomic information in the form of a nucleotide sequence, it will be understood that the methods and systems for compression can be implemented for other genomic or proteomic sequences as well, albeit with a few variations, as will be understood by a person skilled in the art.

Genome sequencing information is generated by High Throughput Sequencing (HTS) machines in the form of sequences of nucleotides (a. k. a. bases) represented by strings of letters from a defined vocabulary. The smallest vocabulary is represented by five symbols: {A, C, G, T, N} representing the 4 types of nucleotides present in DNA namely Adenine, Cytosine, Guanine, and Thymine. In RNA Thymine is replaced by Uracil (U). N indicates that the sequencing machine was not able to call any base and so the real nature of the position is undetermined. In case the IUPAC ambiguity codes are adopted by the sequencing machine, the alphabet used for the symbols is (A, C, G, T, U, W, S, M, K, R, Y, B, D, H, V, N or -).

The nucleotides sequences produced by sequencing machines are called "**reads**". Sequence reads can be between a few dozens to several thousand nucleotides long. Some technologies produce sequence reads in **pairs** where one read is from one DNA strand and the second is from the other strand. In genome sequencing the term **coverage** is used to express the level of redundancy of the sequence data with respect to a **reference sequence.** For example, to reach a coverage of 30x on a human genome (3.2 billion bases long) a sequencing machine shall produce a total of 30 x 3.2 billion bases so that in average each position in the reference is "covered" 30 times.

Throughout this disclosure, a **reference sequence** is any sequence on which the nucleotides sequences produced by sequencing machines are aligned/mapped. One example of sequence could actually be a **reference genome,** a sequence assembled by scientists as a representative example of a species' set of genes. For example GRCh37, the Genome Reference Consortium human genome (build 37) is derived from thirteen anonymous volunteers from Buffalo, New York. However, a reference sequence could also consist of a synthetic sequence conceived to merely improve the compressibility of the reads in view of their further processing.

Sequencing devices can introduce errors in the sequence reads such as
1. Use of a wrong symbol (i.e. representing a different nucleic acid) to represent the nucleic acid actually present in the sequenced sample; this is usually called "substitution error" (mismatch);
2. Insertion in one sequence read of additional symbols that do not refer to any actually present nucleic acid; this is usually called "insertion error";
3. Deletion from one sequence read of symbols that represent nucleic acids that are actually present in the sequenced sample; this is usually called "deletion error";
4. Recombination of one or more fragments into a single fragment which does not reflect the reality of the originating sequence;

The term "coverage" is used in literature to quantify the extent to which a reference genome or part thereof can be covered by the available sequence reads. Coverage is said to be:
- partial (less than 1X) when some parts of the reference genome are not mapped by any available sequence read.
- single (1X) when all nucleotides of the reference genome are mapped by one and only one symbol present in the sequence reads.
- multiple (2X, 3X, NX) when each nucleotide of the reference genome is mapped multiple times.

This invention aims at defining a genomic information representation format where the relevant information is efficiently accessible and transportable and the weight of the redundant information is reduced.

The main aspects of the disclosed invention are:
1 The classification of the sequence reads in different classes according to the results of the alignment with respect to the reference sequences in order to enable selective access to encoded data according to criteria related to the alignment results and to matching accuracy.
2 The decomposition of the sequence read data and metadata into homogeneous layers of in order to obtain distinct information sources with reduced information entropy.
3 The possibility of modeling each separated source with distinct source model adapted to each statistical characteristics including the possibility of changing the source model within each class of reads and layer for each accessible data units (access units). Adoption of the appropriate context adaptive probability models and associated entropy coders according to the statistical properties of each source model.
4 The definition of correspondences and dependencies among the layers to enable selective access to the data without the need to decode all the layers if not all information is needed
5 Coding each sequence data class and associated metadata layers with respect to a reference sequence that can be modified so as to reduce the entropy of data classes and layers information sources. After a first encoding based on a reference, sequence the detected mismatches can be used to "adapt/modify" the reference sequence in order to further reduce the overall information entropy. This process that can be performed iteratively as long as the reduction of information entropy results relevant.

In the following, each of the above aspects will be further described.

### Main file header

### Classification of the sequence reads

The sequence reads generated by sequencing machines are classified by the disclosed invention into five different "classes" according to the results of the alignment with respect to one or more given reference sequences.

When aligning a DNA sequence of nucleotides with respect to a reference sequence five are the possible results:
1. A region in the reference sequence is found to match the sequence read without any error (perfect mapping). Such sequence of nucleotides will be referenced to as "perfectly matching read" or denoted as "Class P".
2. A region in the reference sequence is found to match the sequence read with a number of mismatches constituted by a number of positions in which the sequencing machine was not able to call any base (or nucleotide). Such mismatches are denoted by an "N". Such sequences will be referenced to as "N mismatching reads" or "Class N".
3. A region in the reference sequence is found to match the sequence read with a number of mismatches constituted by a number of positions in which the sequencing machine was not able to call any base (or nucleotide) OR a different base than the one reported in the reference genome has been called. Such type of mismatch is called Single Nucleotide Variation (SNV) or Single Nucleotide Polymorphism (SNP). The sequence will be referenced to as "M mismatching reads" or "Class M".
4. A fourth class is constituted by sequencing reads presenting a mismatch type that includes the same mismatches of class M plus the presence of insertions or deletions (a.k.a. *indels*)*.* Insertions are represented by a sequence of one or more nucleotides not present in the reference, but present in the read sequence. In literature when the inserted sequence is at the edges of the sequence it is referred to as "soft clipped" (i.e. the nucleotides are not matching the reference but are kept in the aligned reads contrarily to "hard clipped" nucleotides which are discarded). Keeping or discarding nucleotides is typically a user's decisions implemented as a configuration of the aligning tool. Deletion are "holes" (missing nucleotides) in the aligned read with respect to the reference. Such sequences will be referenced to as "I mismatching reads" or "Class I".
5. A fifth class includes all reads that do now find any valid mapping on the reference genome according to the specified alignment constraints. Such sequences are said to be Unmapped and belonging to "Class U".

The remaining unmapped reads with respect to a reference sequence can be assembled into a single sequence using de-novo assembly algorithms. Once a newly assembled reference sequence has been created unmapped reads can be further mapped with respect to it and be classified in one of the 4 classes P, N, M and I.

### Decomposition of the information necessary to represent sequence reads into layers of descriptors

Once the classification of reads is completed with the definition of the Classes, further processing consists in defining a set of distinct syntax elements which represent the remaining information enabling the reconstruction of the DNA read sequence when represented as being mapped on a given reference sequence. The data structure of these syntax elements requires the storage of global parameters and metadata to be used by the decoding engine. These data are structured in a ***main header*** described in the table below.

**Table 1- Main Header structure.**

| **Element** | **Type** | **Description** |
|---|---|---|
| **Unique ID** | Byte array | Unique identifier for the encoded content |
| **Version** | Byte array | Major + Minor version of the encoding algorithm |
| **Header Size** | Integer | Size in bytes of the entire encoded content |
| **Reads Length** | Integer | Size of reads in case of constant reads length. A special value (e.g. 0) is reserved for variable reads length |
| **Ref count** | Integer | Number of reference sequences used |
| **Access Units counters** | Byte array (e.g. integers) | Total Number of encoded Access Units per reference sequence |
| **Ref ids** | Byte array | Unique identifiers for reference sequences |
| **Master index table** | Byte array (e.g. integers) | This is a multidimensional array supporting random access to Access Units. |
| *Alignment positions of first read in each block* (*Access Unit).* | | |
| *I.e. smaller position of the first read on the reference genome per each block of the 4 classes* | | |
| *1 per pos class (4) per reference* | | |

A DNA segment referred to a given reference sequence can be fully expressed by:
- The starting position on the reference sequence (pos)
- A flag signaling if the read has to be considered as a reverse complement versus the reference (rcomp).
- A distance, to the mate pair in case of paired reads (pair).
- The value of the read length in case of the sequencing technology produces variable length reads (len). In case of constant reads length the read length associated to each reads can obviously be omitted and can be stored in the main file header.
- For each mismatch:
   o Mismatch position (*nmis* for class N, *snpp* for class M, and *indp* for class I)
   o Mismatch type (not present in class N, *snpt* in class M, *indt* in class I)
- Flags indicating specific characteristics of the sequence read such as
   o template having multiple segments in sequencing
   o each segment properly aligned according to the aligner
   o unmapped segment
   o next segment in the template unmapped
   o signalization of first or last segment
   o quality control failure
   o PCR or optical duplicate
   o secondary alignment
   o supplementary alignment
- Optional soft clipped nucleotides string when present (*indc* in class I)

This classification creates groups of descriptors (syntax elements) that can be used to univocally represent genome sequence reads. The table below summarizes the syntax elements needed for each class of aligned reads.

**Table 2 - Defined layers per class of data.**

| | P | N | M | I |
|---|---|---|---|---|
| pos | X | X | X | X |
| pair | X | X | X | X |
| rcomp | X | X | X | X |
| flags | X | X | X | X |
| rlen | X | X | X | X |
| nmis | | X | | |
| snpp | | | X | |
| snpt | | | X | |
| indp | | | | X |
| indt | | | | X |
| indc | | | | X |

Reads belonging to class P are characterized and can be perfectly reconstructed by only a position, a reverse complement information and an offset between mates in case they have been obtained by a sequencing technology yielding mated pairs, some flags and a read length.

The next section further details how these descriptors are defined.

### Position descriptor layer

In the position (**pos**) layer only the mapping position of the first encoded read is stored as absolute value on the reference sequence. All the other position descriptors assume a value expressing the difference with respect to the previous position. Such modeling of the information source defined by the sequence of read position descriptors is in general characterized by a reduced entropy particularly for sequencing processes generating high coverage results.

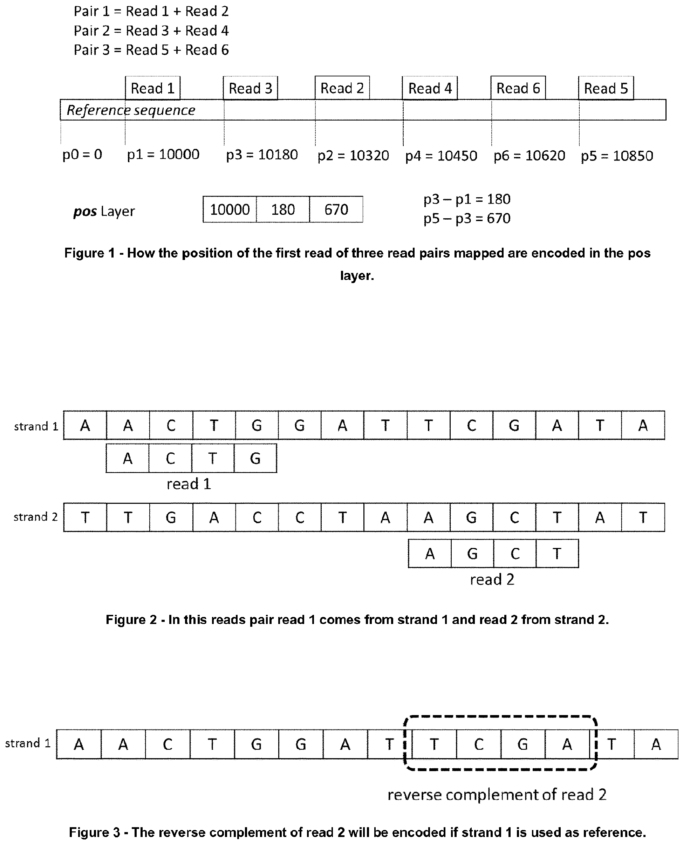

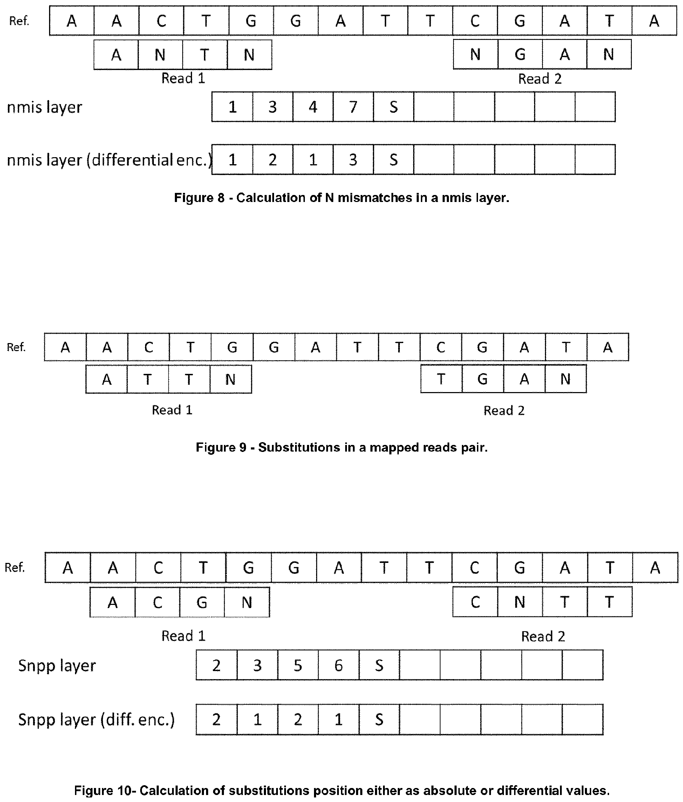

For example, figure 1 shows how after describing the starting position of the first alignment as position "10000" on the reference sequence, the position of the second read starting at position 10180 is described as "180". With high coverages (> 50x) most of the descriptors of the position vector will present very high occurrences of low values such as 0 and 1 and other small integers. Figure 9 shows how the positions of three read pairs are described in a **pos** Layer.

### Reverse complement descriptor layer

Each read of the read pairs produced by sequencing technologies can be originated from either genome strands of the sequenced organic sample. However, only one of the two strands is used as reference sequence. Figure 2 shows how in a reads pair one read (read 1) can come from one strand and the other (read 2) can come from the other.

When the strand 1 is used as reference sequence, read 2 can be encoded as *reverse complement* of the corresponding fragment on strand 1. This is shown in figure 3.

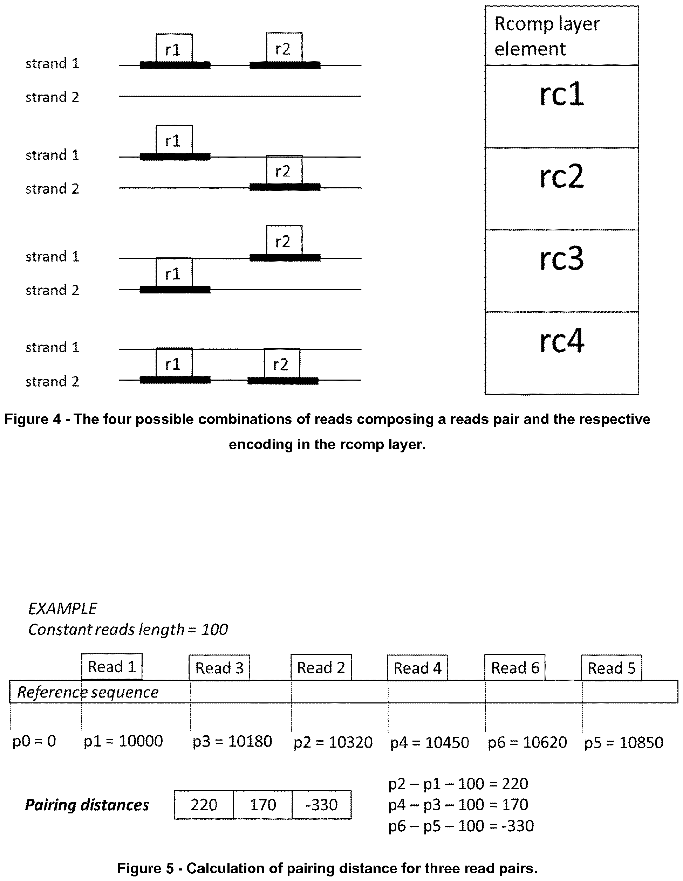

In case of coupled reads, four are the possible combinations of direct and reverse complement mate pairs. This is shown in figure 4. The rcomp layer encodes the four possible combinations .

The same encoding is used for the reverse complement information of reads belonging to classes N, M, P and I. In order to enable selective access to the different data classes, the reverse complement information of reads belonging to the four classes are encoded in different layers as depicted in Table 2.

### Pairing information descriptor layer

The pairing descriptor is stored in the pair layer. Such layer stores descriptors encoding the information needed to reconstruct the originating reads pairs when the employed sequencing technology produces reads by pairs. Although at the date of the disclosure of the invention the vast majority of sequencing data is generated by using a technology generating paired reads, it is not the case of all technologies. This is the reason for which the presence of this layer is not necessary to reconstruct all sequencing data information if the sequencing technology of the genomic data considered does not generate paired reads information.

### Definitions:

- **mate pair:** read associated to another read in a read pair (e.g. Read 2 is the mate pair of Read 1 in the previous example)
- **pairing distance:** number of nucleotide positions on the reference sequence which separate one position in the first read (pairing anchor, e.g. last nucleotide of first read) from one position of the second read (e.g. the first nucleotide of the second read)
- **most probable pairing distance (MPPD):** this is the most probable pairing distance expressed in number of nucleotide positions.
- **position pairing distance (PPD):** the PPD is a way to express a pairing distance in terms of the number of reads separating one read from its respective mate present in a specific position descriptor layer.
- **most probable position pairing distance (MPPPD):** is the most probable number of reads separating one read from its mate pair present in a specific position descriptor layer.
- **position pairing error (PPE):** is defined as the difference between the MPPD or the MPPPD and the actual position of the mate.
- **pairing anchor:** position of first read last nucleotide in a pair used as reference to calculate the distance of the mate pair in terms of number of nucleotide positions or number of read positions.

Figure 5 shows how the pairing distance among read pairs is calculated.

The pair descriptor layer is the vector of pairing errors calculated as number of reads to be skipped to reach the mate pair of the first read of a pair with respect to the defined decoding pairing distance.

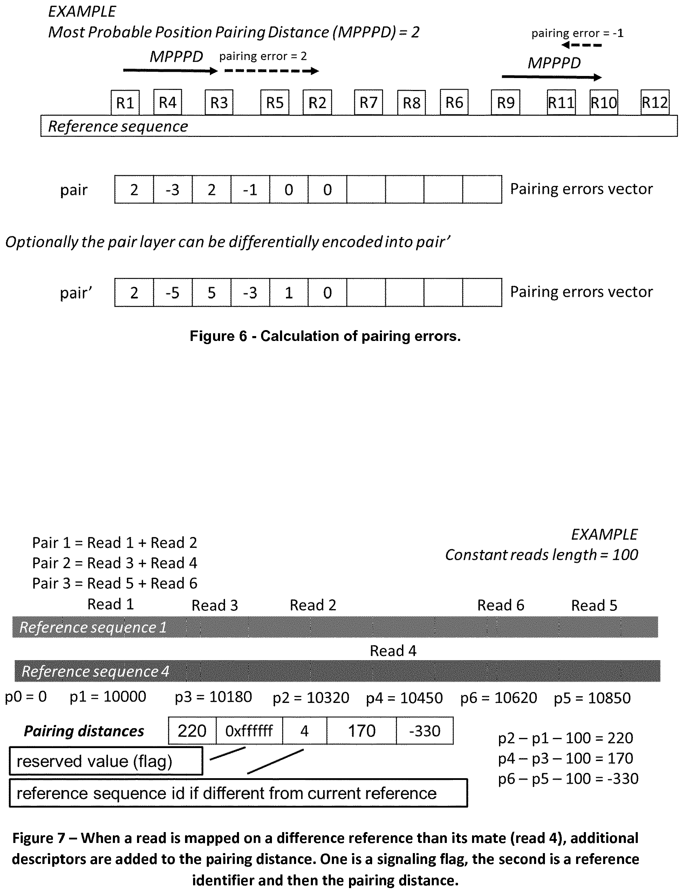

Figure 6 shows an example of how pairing errors are calculated, both as absolute value and as differential vector (characterized by lower entropy for high coverages).

The same descriptors are used for the pairing information of reads belonging to classes N, M, P and I. In order to enable the selective access to the different data classes, the pairing information of reads belonging to the four classes are encoded in different layer as depicted in.

### Pairing information in case of reads mapped on different reference sequences

In the process of mapping sequence reads on a reference sequence it is not uncommon to have the first read in a pair mapped on one reference sequence (e.g. chromosome 1) and the second on a different reference sequence (e.g. chromosome 4). In this case the pairing information described above has to be integrated by additional information related to the reference sequence used to map one of the reads. This is achieved by coding
1. A reserved value (flag) indicating that the pair is mapped on two different sequences (different values indicate if read1 or read2 are mapped on the sequence that is not currently encoded)
2. An unique reference identifier referring to the reference identifiers encoded in the main header structure as described Table 1.
3. The third element contains the mapping information on the reference identified at point 2 and expressed as offset with respect to the last encoded position.

The figure 7 provides an example of this scenario.

In figure 7, since Read 4 is not mapped on the currently encoded reference sequence, the genomic encoder signals this information by crafting additional descriptors in the pair layer. In the example shown below Read 4 of pair 2 is mapped on reference no. 4 while the currently encoded reference is no. 1. This information is encoded using 3 components:
1) One special reserved value is encoded as pairing distance (in this case 0xffffff)
2) A second descriptor provides a reference ID as listed in the main header (in this case 4)
3) The third element contains the mapping information on the concerned reference (170).

### Mismatch descriptors for class N reads

Class N includes all reads in which only mismatches constituted by "N" are present at the place of an A, C, G or T base call. All other bases of the read perfectly match the reference sequence.

Figure 8 shows how:
the positions of "N" in read 1 are coded as
   - absolute position in read 1 or
   - as differential position with respect to the previous "N" in the same read.
the positions of "N" in read 2 are encoded as
   - absolute position in read 2 + read 1 length or
   - differential position with respect to the previous N

In the **nmis** layer, the coding of each reads pair is terminated by a special "separator" symbol.

Figure 8 shows how "N" mismatches (where, at a given mapping position, a "N" is present in a read instead of an actual base in the reference sequence) are encoded only as a the position of the mismatch
1. with respect to the beginning of the read or
2. with respect to the previous mismatch (differential encoding)

### Descriptors coding Substitutions (Mismatches or SNPs), Insertions and Deletions

A substitution is defined as the presence, in a mapped read, of a different nucleotide base with respect to the one that is present in the reference sequence at the same position.

Figure 9 shows examples of substitutions in a mapped read pair. Each substitution is encoded as "position" (snpp layer) and "type" (snpt layer). Depending on the statistical occurrence of substitutions, insertion or deletion, different source models of the associated descriptors can be defined and the generated symbols coded in the associated layer.

### Source model 1: Substitutions as Positions and Types

### Substitutions Positions Descriptors

A substitution position is calculated like the values of the nmis layer, i.e.

In read 1 substitutions are encoded
- as absolute position in read 1 or
- as differential position with respect to the previous substitution in the same read In read 2 substitutions are encoded
- as absolute position in read 2 + read 1 length or
- as differential position with respect to the previous substitution

Figure 10 shows how substitutions (where, at a given mapping position, a symbol in a read is different from the symbol in the reference sequence) are coded as
1. the position of the mismatch
   ▪ with respect to the beginning of the read or
   ▪ with respect to the previous mismatch (differential encoding)
2. the type of mismatch represented as a code calculated as described in Figure 10

In the snpp layer, the coding of each reads pair is terminated by a special "separator" symbol.

### Substitutions Types Descriptors

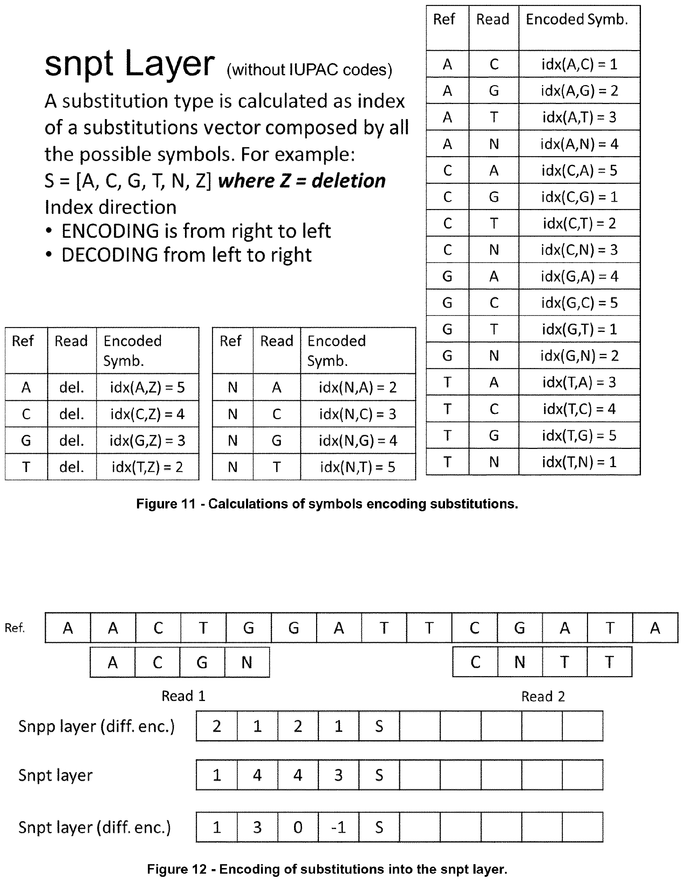

For class M (and I as described in the next sections), mismatches are coded by an index (moving from right to left) from the actual symbol present in the reference to the corresponding substitution symbol present in the read {A, C, G, T, N, Z}. For example if the aligned read presents a C instead of a T which is present at the same position in the reference, the mismatch index will be denoted as "4". The decoding process reads the encoded syntax element, the nucleotide at the given position on the reference and moves from left to right to retrieve the decoded symbol. E.g. a "2" received for a position where a G is present in the reference will be decoded as "N". Figure 11 shows all the possible substitutions and the respective encoding symbols. Obviously different and context adaptive probability models can be assigned to each substitution index according to the statistical properties of each substitution type for each data class to minimize the entropy of the descriptors.

In case of adoption of the IUPAC ambiguity codes the substitution mechanism results to be exactly the same however the substitution vector is extended as: S = {A, C, G, T, N, Z, M, R, W, S, Y, K, V, H, D, B}.

Figure 12 provides an example of encoding of substitutions types in the ***snpt*** layer.

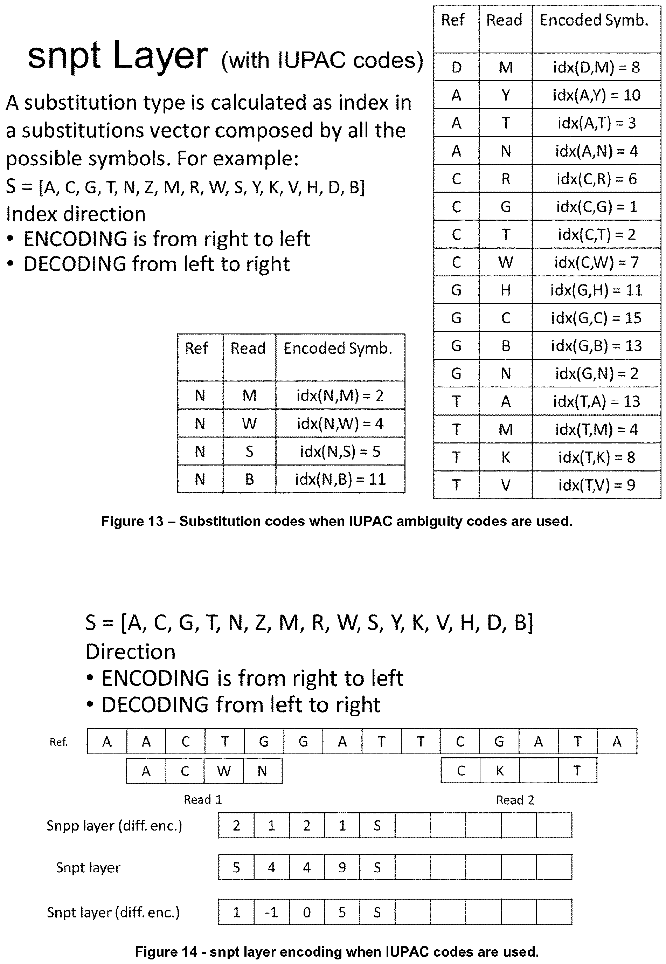

Some examples of substitutions encoding when IUPAC ambiguity codes are adopted are provided in Figure 13. A further example of substitution indexes is provided in Figure 14.

### Cooding of insertions and deletions

For class I, mismatches and deletions are coded by an indexes (moving from right to left) from the actual symbol present in the reference to the corresponding substitution symbol present in the read: {A, C, G, T, N, Z}. For example if the aligned read presents a C instead of a T present at the same position in the reference, the mismatch index will be "4". In case the read presents a deletion where a A is present in the reference, the coded symbol will be "5". The decoding process reads the coded syntax element, the nucleotide at the given position on the reference and moves from left to right to retrieve the decoded symbol. E.g. a "3" received for a position where a G is present in the reference will be decoded as "Z".

Inserts are coded as 6, 7, 8, 9, 10 respectively for inserted A, C, G, T, N.

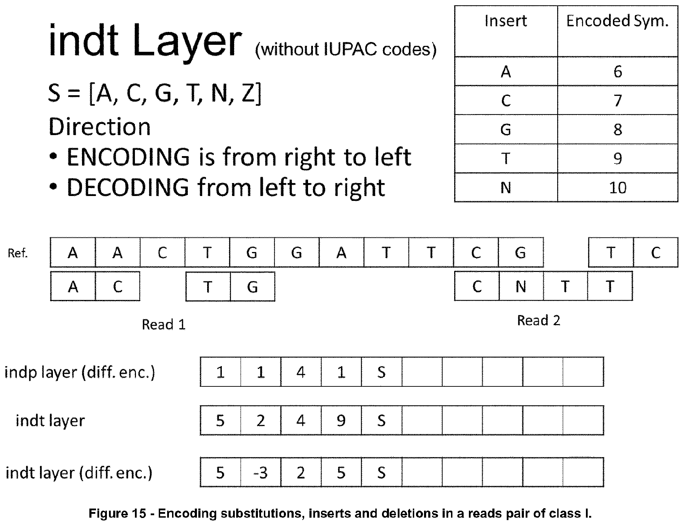

Figure 15 shows an example of how to encode substitutions, inserts and deletions in a reads pair of class I. In order to support the entire set of IUPAC ambiguity codes, the substitution vector S= {A, C, G, T, N, Z} shall be replaced by S = {A, C, G, T, N, Z, M, R, W, S, Y, K, V, H, D, B} as described in the previous paragraph for mismatches.

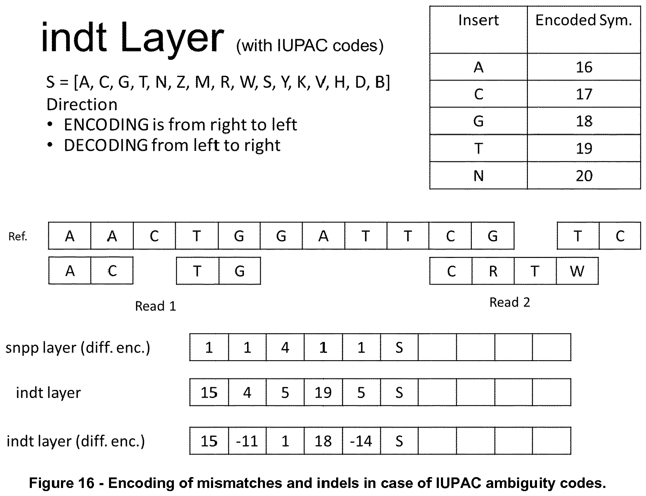

In this case the insertion codes need to have different values, namely 16, 17, 18, 19, 20 in case the substitution vector has 16 elements. The mechanism is illustrated in Figure 16.

### Source model 2: One layer per substitution type and indels

For some data statistics a different coding model from the one described in the previous section can be developed for substitutions and indels resulting into a source with lower entropy. Such coding model is an alternative to the techniques described above for mismatches only and for mismatches and indels.

In this case one data layer is defined for each possible substitution symbol (5 without IUPAC codes, 16 with IUPAC codes), plus one layer for deletions and 4 more layers for insertions. For simplicity of the explanation, but not as a limitation for the application of the model, the following description will focus on the case where no IUPAC codes are supported.

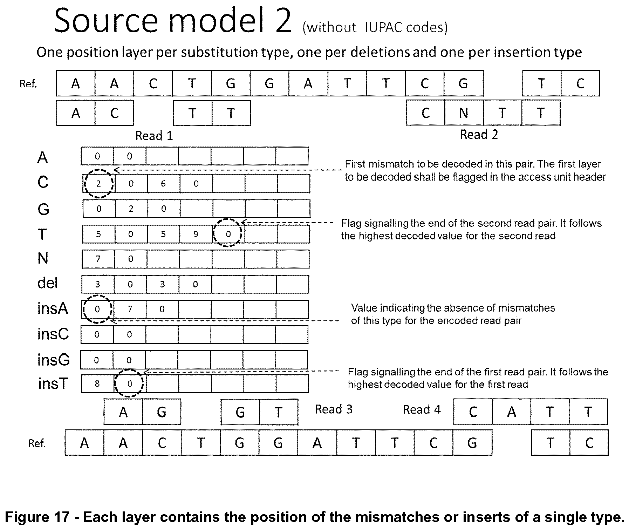

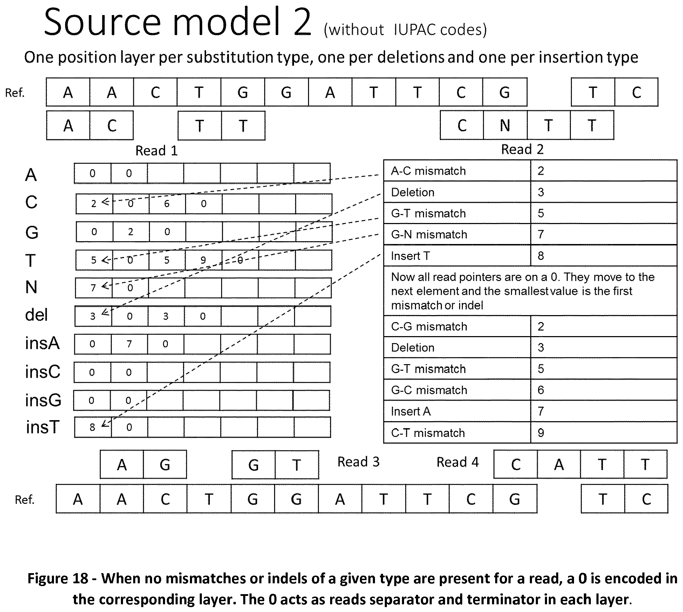

Figure 17 shows how each layer contains the position of the mismatches or inserts of a single type. If no mismatches or inserts for that type is present in the encoded read pair, a 0 is encoded in the corresponding layer. To enable the decoder to start the decoding process for the layers described in this section, the header of each access units contains a flag signaling the first layer to be decoded. In the example of figure 18 the first element to be decoded is position 2 in the C layer. When no mismatches or indels of a given type are present in a read pair, a 0 is added to the corresponding layers. On the decoding side, when the decoding pointer for each layer points to a value of 0, the decoding process moves to the next read pair.

### Encoding additional signaling flags

Each data class introduced above (P, M, N, I) may require the encoding of additional information on the nature of the encoded reads. This information may be related for example to the sequencing experiment (e.g. indicating a probability of duplication of one read) or can express some characteristic of the read mapping (e.g. first or second in pair). In the context of this invention this information is encoded in a separate layer for each data class. The main advantage of such approach is the possibility to selectively access this information only in case of need and only in the required reference sequence region. Other examples of the use of such flags are:
- read paired
- read mapped in proper pair
- read or mate unmapped
- read or mate from reverse strand
- first/second in pair
- not primary alignment
- read fails platform/vendor quality checks
- read is PCR or optical duplicate
- supplementary alignment

### Adaptation of the reference sequences

The mismatches encoded for classes N, M and I can be used to create "modified references" to be used to re-encode reads in the N, M or I layer (with respect to the first reference sequence, R0) as p reads with respect to the "adapted" genome R1. For example if we denote with r_in^M the ith read of class M containing mismatches with respect to the reference genome n, then after "adaptation" we could have r_in^M = r_(i(n+1))^P with A(Refn)=Refn+1 where A is the transformation from reference sequence n to reference sequence n + 1.

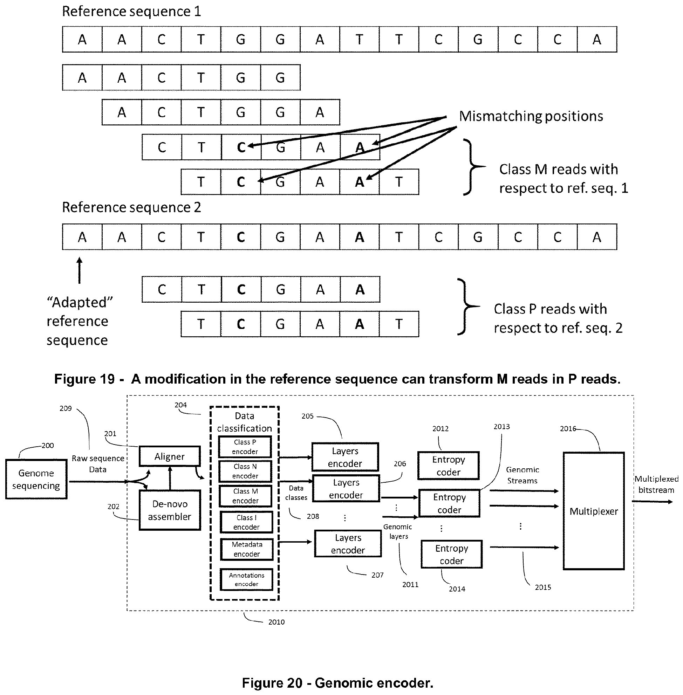

Figure 19 shows how reads containing mismatches (M reads) with respect to reference sequence 1 (RS1) can be transformed into perfectly matching reads (P reads) with respect to reference sequence 2 (RS2) obtained from RS1 by modifying the mismatching positions. This transformation can be expressed as
RS2 = A(RS1)

If the expression of transformation A which goes from RS1 to RS2 requires less bits of the expression of the mismatches present in the M reads, this encoding method results in a smaller information entropy and therefore better compression.

### Source models, entropy coders and coding modes

For each layer of the genomic data structure disclosed in this invention different coding algorithms may be adopted according to the specific features of the data or metadata carried by the layer and its statistical properties. The "coding algorithm" has to be intended as the association of a specific "source model" of the descriptor with a specific "entropy coder". The specific "source model" can be specified and selected to obtain the most efficient coding of the data in terms of minimization of the source entropy. The selection of the entropy coder can be driven by coding efficiency considerations and/or probability distribution features and associated implementation issues. Each selection of a specific coding algorithm will be referred to as "coding mode" applied to an entire "layer".

Each "source model" associated to a coding mode is characterized by:
- The definition of the syntax elements emitted by each source (e.g. reads position, reads pairing information, mismatches with respect to a reference sequence etc.)
- The definition of the associated probability model.
- The definition of the associated entropy coder.

### Further advantages

This classification permits the implementation of efficient coding modes exploiting the lower information source entropy characterizing by modelling the sequences of syntax elements by single separate data sources (e.g. distance, position, etc.).

Another advantage of the invention is the possibility to access only the subset of type of data of interest. For example one of the most important application in genomics consists in finding the differences of a genomic sample with respect to a reference (SNV) or a population (SNP). Today such type of analysis requires the processing of the complete sequence reads whereas by adopting the data representation disclosed by the invention the mismatches are already isolated into one to three data classes only (depending on the interest in considering N codes and indels).

A further advantage is the possibility of performing efficient transcoding from data and metadata compressed with reference to a specific "reference sequence" to another "reference sequence" when a new "reference sequence" is published or when re-mapping is performed on the already mapped data (e.g. using a different mapping algorithm).

Figure 20 shows an encoding apparatus 207 according to the principles of this invention. The encoding apparatus 207 receives as input a raw sequence data 209, for example produced by a genome sequencing apparatus 200. Genome sequencing apparatus 200 are known in the art, like the Illumina HiSeq 2500 or the Thermo-Fisher Ion Torrent devices. The raw sequence data 209 is fed to an aligner unit 201, which prepares the sequences for encoding by aligning the reads to a reference sequence. Alternatively, a de-novo assembler 202 can be used to create a reference sequence from the available reads by looking for overlapping prefixes or suffixes so that longer segments (called "contigs") can be assembled from the reads. After having been processed by a de-novo assembler 202, reads can be mapped on the obtained longer sequence. The aligned sequences are then classified by data classification module 204. The data classes 208 are then fed to layers encoders 205-207. The genomic layers 2011 are then fed to arithmetic encoders 2012-2014 which encode the layers according to the statistical properties of the data or metadata carried by the layer. The result is a genomic stream 2015.

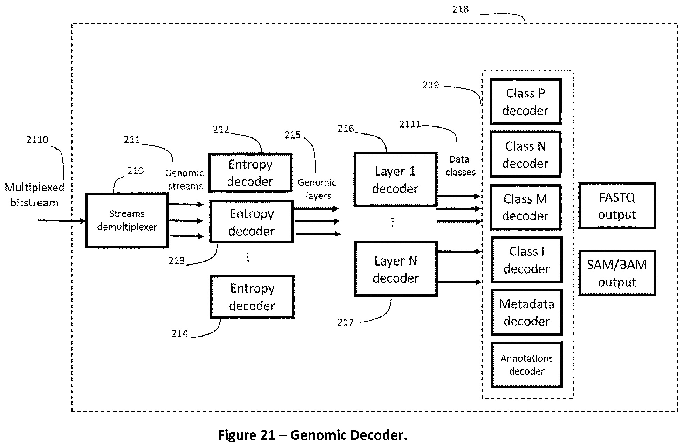

Figure 21 shows a decoding apparatus 218 according to the principles of this disclosure. A decoding apparatus 218 receives a multiplexed genomic bitstream 2110 from a network or a storage element. The multiplexed genomic bitstream 2110 is fed to a demultiplexer 210, to produce separate streams 211 which are then fed to entropy decoders 212-214, to produce genomic layers 215. The extracted genomic layers are fed to layer decoders 216-217 to further decode the layers into classes of data. Class decoders 219 further process the genomic descriptors and merge the results to produce uncompressed reads of sequences, which can then be further stored in the formats known in the art, for instance a text file or zip compressed file, or FASTQ or SAM/BAM files.

Class decoders 219 are able to reconstruct the original genomic sequences by leveraging the information on the original reference sequences carried by one or more genomic streams. In case the reference sequences are not transported by the genomic streams they must be available at the decoding side and accessible by the class decoders.

The inventive techniques herewith disclosed may be implemented in hardware, software, firmware or any combination thereof. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and similar devices.

## Claims

1. A computer-implemented method for the compression of genome sequence data produced by a sequencing machine, said genome sequence data comprising reads of sequences of nucleotides,
said method comprising the steps of:
aligning said reads to one or more reference sequences thereby creating aligned reads, classifying said aligned reads according to matching accuracy degrees with the one or more reference sequences thereby creating classes of aligned reads;
encoding said classified and aligned reads as a multiplicity of syntax elements,
wherein encoding said classified aligned reads as a multiplicity of syntax elements comprises selecting said syntax elements according to said classes of aligned reads.

2. The method of claim 1, wherein said syntax elements comprise a position along the reference sequence, a distance between two positions on the reference sequence and information indicating if sequence reads are reverse complemented.

3. The method of claim 2, wherein said syntax elements further comprise the position of a variant with respect to the reference sequence, the type of variant, the position of a deletion with respect to the reference sequence, the position of one or more symbols not present in the reference sequence, but present in the aligned reads, the type of insertion at a given position.

4. The method of claim 1, wherein the encoding of said classified aligned reads as a multiplicity of syntax elements is adapted according to the specific features of the data or metadata represented by said syntax elements.

5. The method of claim 4, wherein the encoding of said classified aligned reads as multiplicity of syntax elements is further adapted according to the statistical properties of the data or metadata represented by said syntax elements.

6. The method of claim 5, wherein the encoding of said classified aligned reads as multiplicity of syntax elements associates a specific source model and a specific entropy coder to each syntax element.

7. The method of claim 5, wherein said entropy coder is a context adaptive arithmetic coder.

8. The method of claim 1, wherein said sequence reads perfectly match on one or more reference sequences.

9. The method of claim 1, wherein said sequence reads contain only mismatches where an "N" symbol is present instead of an actual nucleotide or aminoacid symbol with respect to one or more reference sequences.

10. The method of claim 1, wherein said sequence reads contain any type of mismatch in the form of substitution of symbols with respect to one or more reference sequences.

11. The method of claim 1, wherein said sequence reads contain mismatches and insertion or deletions with respect to one or more reference sequences.

12. The method of claim 1, wherein said sequence reads contain mismatches, insertion or deletions and soft clipped symbols with respect to one or more reference sequences.

13. The method of claim 1, wherein said sequence reads cannot be mapped with respect to the one or more reference sequences.

14. The method of claim 1, wherein said sequence reads are further classified into different classes according to the number of mismatches contained by each sequence read.

15. A computer-implemented method for the decompression of a compressed genomic stream, said method comprising the steps of:
parsing said compressed genomic stream into syntax elements,
expanding said syntax elements into classified reads of sequences of nucleotides,
selectively decoding said classified reads of sequences of nucleotides on one or more reference sequences so as to produce uncompressed reads of sequences of nucleotides.

16. A computer-implemented method for the classification of genome sequence data produced by a sequencing machine, said genome sequence data comprising sequences of nucleotides "bases", said classification being performed according to a reference sequence, said method comprising the steps of:
identifying class P sequences, comprising matching regions in the reference sequence without mismatches;
identifying class N sequences, comprising matching regions in the reference sequence with a number of mismatches represented by positions where the sequencing machine was not able to call any "base" identifying class M sequences, comprising matching regions in the reference sequence with a number of mismatches represented by positions where the sequencing machine was not able to call any base or it called a different base than the reference sequence identifying class I sequences, comprising the same mismatches of class M plus the presence of insertions or deletions,
identifying class U sequences comprising all reads that do not find any valid mapping on the reference sequence.

17. A genomic encoder 2010 for the compression of genome sequence data 209, said genome sequence data 209 comprising reads of sequences of nucleotides,
said genomic encoder 2010 comprising:
an aligner unit 201, configured to align said reads to one or more reference sequences thereby creating aligned reads,
a data classification unit 204, configured to classify said aligned reads according to matching accuracy degrees with the one or more reference sequences thereby creating classes of aligned reads;
one or more encoding units 205-207, configured to encode said classified aligned reads as syntax elements by selecting said syntax elements according to said classes of aligned reads.

18. A genomic decoder 218 for the decompression of a compressed genomic stream 211 said genomic decoder 218 comprising:
parsing means 210, 212-214 configured to parse said compressed genomic stream into syntax elements 215,
one or more decoders 216-217, configured to decode the syntax elements into classified reads of sequences of nucleotides 2111,
genomic data classes decoders 213 configured to selectively decode said classified reads of sequences of nucleotides on one or more reference sequences so as to produce uncompressed reads of sequences of nucleotides.

19. The genomic decoder of claim 18, wherein the one or more reference sequences are stored in the compressed genome stream 211.

20. The genomic decoder of claim 18, wherein the one or more reference sequences are provided to the decoder via an out of band mechanism.

21. The genomic decoder of claim 18, wherein the one or more reference sequences are built at the decoder.

22. A computer-readable medium comprising instructions that when executed cause at least one processor to perform the method of any one of claims 1 to 16.
